Europäisches Patentamt

European Patent Office

Office européen des brevets

(19)

(11) Veröffentlichungsnummer : **0 007 133**
**B1**

(12) **EUROPÄISCHE PATENTSCHRIFT**

(45) Veröffentlichungstag der Patentschrift :
16.02.83

(21) Anmeldenummer : **79200362.6**

(22) Anmeldetag : **04.07.79**

(51) Int. Cl.³ : **C 12 M   1/12//** C12G3/00,
C12P7/48

(54) **Fermenter.**

(30) Priorität : **19.07.78 CH 7774/78**

(43) Veröffentlichungstag der Anmeldung :
**23.01.80 Patentblatt 80/02**

(45) Bekanntmachung des Hinweises auf die Patenterteilung : **16.02.83 Patentblatt 83/07**

(84) Benannte Vertragsstaaten :
**AT CH DE FR**

(56) Entgegenhaltungen :
**US A 3 223 595**
**US A 3 321 086**
**US A 3 915 802**
**CHEMICAL ABSTRACTS, Band 89, Nr. 3, 17. Juli
1978, Zusammenfassung Nr. 22284k, Seite 506,
Columbus, Ohio, US, A. MARGARITIS et al. :
« The rotor-fermentor. I. Description of the apparatus, power requirements, and mass transfer
characteristics »**
**CHEMICAL ABSTRACTS, Band 89, Nr. 3, 17. Juli
1978, Zusammenfassung Nr. 22285m, Seite 507,
Columbus, Ohio, US, A. MARGARITIS et al. :
« The rotor-fermentor. II. Application to ethanol
fermentation »**

(73) Patentinhaber : **Chemap AG
Alte Landstrasse 415
CH-8708 Männedorf (CH)**

(72) Erfinder : **Müller, Felix, Dr.
Bahnhofstrasse 46
CH-8712 Stäfa (CH)**

(74) Vertreter : **Herrmann, Peter
Chemap AG Alte Landstrasse 415
CH-8708 Männedorf (CH)**

Jouve, 18, rue St-Denis, 75001 Paris, France

## Fermenter

Gegenstand der Erfindung ist ein Fermenter zur Züchtung von Mikroorganismen, unter gleichzeitiger Abtrennung und Entfernung der entstandenen Stoffwechselprodukte mittels Membranfiltration im Innern eines zylindrischen Fermenters. Die Abtrennung der Stoffwechselprodukte von Mikroorganismen nach der Fermentierung zur Gewinnung von insbesondere Essig, Wein, Antibiotika, wird durch filtration über Filter, unter Zugabe von Filterhilfsmitteln in Anschwemmfiltern oder nach Aufgabe einer Precoatschicht über Trommeldrehfilter durchgeführt. Solche Filtrationen können nur im Chargenbetrieb erfolgen. Auch bei der Fermentierung der genannten Stoffwechselprodukte wird vorwiegend diskontinuierlich gearbeitet.

Der Einsatz der Ultrafiltration zur Abtrennung von Fermentationsprodukten ist nach Michaels « Ultrafiltration » Booklet No. 905, AMICON Corp., March 1968, p. 22 bekannt. Aus Biotechnology and Bioengineering 20, 709-753 (1978) ist ein zylindrischer Fermenter bekannt, der zur Abtrennung und Entfernung von Fermentationsprodukten ein Membranfilter im Innern aufweist. Dieser sogenannte Rotorfermenter besteht aus drei Teilen, einem Druckbehälter als Fermenter, einem oberen Filtratbehälter und einer rotierbaren zentralen Membranfiltereinheit. Das Konzentrat wird ausserhalb des Fermenters über ein Entgasungsgefäss gepumpt.

Die Nachteile der bekannten Apparaturen sind vielfältig. Durch ausserhalb der Reaktionsbehälter angebrachte Membranfilter sind zusätzliche Pumpen, Rohrleitungen und Armaturen erforderlich. Die erforderlichen Pumpen haben den Nachteil, dass Mikroorganismen, insbesondere Pilze, leicht zerstört werden. Pumpen sind ebenfalls, wie Messonden und Rohrabschnitte, in denen diese befestigt sind, permanente Quellen für Kontaminationen durch fremde Mikroorganismen. Empfindliche Mikroorganismen, wie hochgezüchtete Essigbakterien, die auch den kürzesten Sauerstoffunterbruch nicht vertragen, müssten auf dem Weg zwischen Austritt aus dem Fermenter und Wiedereintritt, um die Absterbensrate so niedrig wie möglich zu halten, zusätzlich begast werden.

Aufgabe der im Anspruch 1 angebenen Erfindung ist es nun, eine Apparatur zu schaffen, die die aufgezeigten Mängel beseitigt.

Die Erfindung soll anhand von Zeichnungen näher erläutert werden.

Es zeigen :

Figur 1 einen Längsschnitt durch den Fermenter,

Figur 2 einen Querschnitt durch das Leitrohr der Fig. 1,

Figur 3 einen Längsschnitt durch eine Variante des Fermenters,

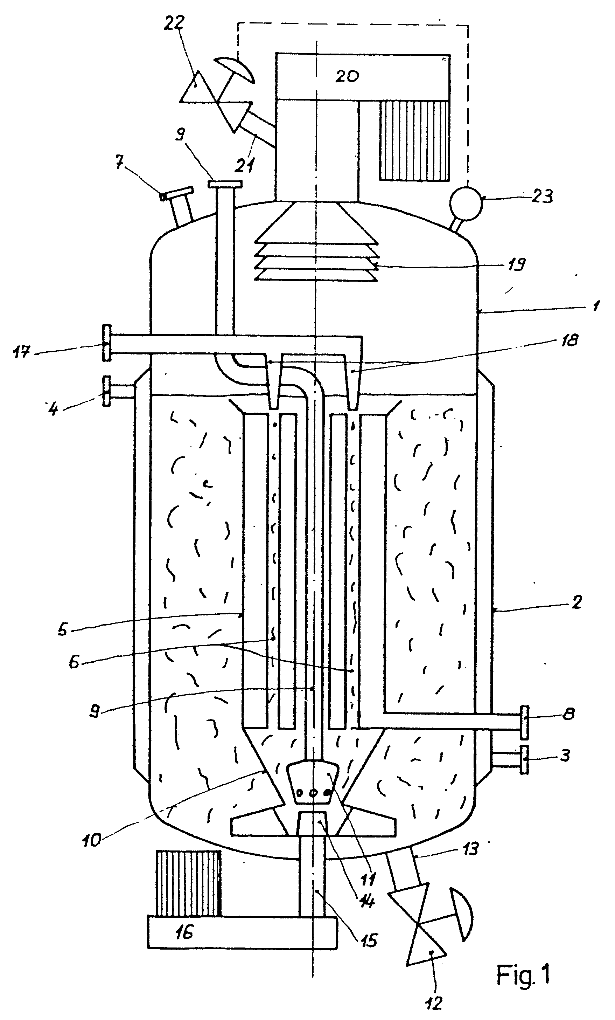

Der im Längsschnitt der Fig. 1 gezeigte Fermenter besteht aus einem Behälter 1 mit einem Doppelmantel 2, mit Anschlussstutzen 3 für den Eingang und Stutzen 4 für den Ausgang des Heiz- bzw. Kühlmediums. Im Innern des Behälters 1 befindet sich ein Leitrohr 5, in welchem im Handel befindliche Membranfilterrohre 6 angebracht sind. Der Zwischenraum zwischen den Membranfilterrohren 6 dient zur Aufnahme des Permeates, dessen einziger Ausgang das Sammelrohr 8 bildet, das durch die Wand des Fermenterkessels 1 hindurchführt. Das Substrat wird über die Leitung 7 im Deckel des Fermenters zugeführt. Durch das Zentrum des Leitrohres 5 führt das Hauptbegasungsrohr 9, das im Konus 10 des Leitrohres 5 in Form eines Verteilerkörpers 11 endet. Im Boden des Fermenters 1 ist ein Ablassventil 12 unterhalb eines Stutzens 13 angebracht. Eine Umwälzturbine 14 wird über eine Welle 15 mittels Elektromotor 16 angetrieben. Im oberen Teil des Fermenters 1 befindet sich eine weitere Luftzufuhrleitung 17, die über Abzweigungsrohre 18 in die Membranfilterrohre 6 mündet. Jedem Membranfilterrohr 6 ist ein Luftzufuhrrohr 18 zugeordnet. Im Kopf des Fermenters ist ein mechanischer Schaumabscheider 19 installiert, der mittels eines Elektromotors 20 angetrieben wird und eine Abgasleitung 21 aufweist. Zur Drosselung des Abgasstromes und zur Druckerhöhung im Fermenterkessel ist über ein Manometer 23 ein Steuerventil 22 eingebaut.

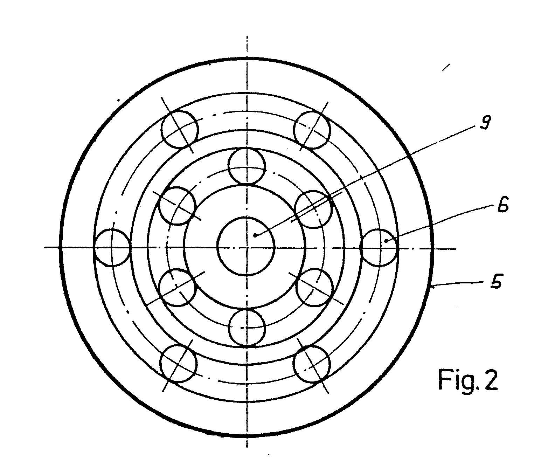

Fig. 2 zeigt den Querschnitt durch das Leitrohr des Fermenters.

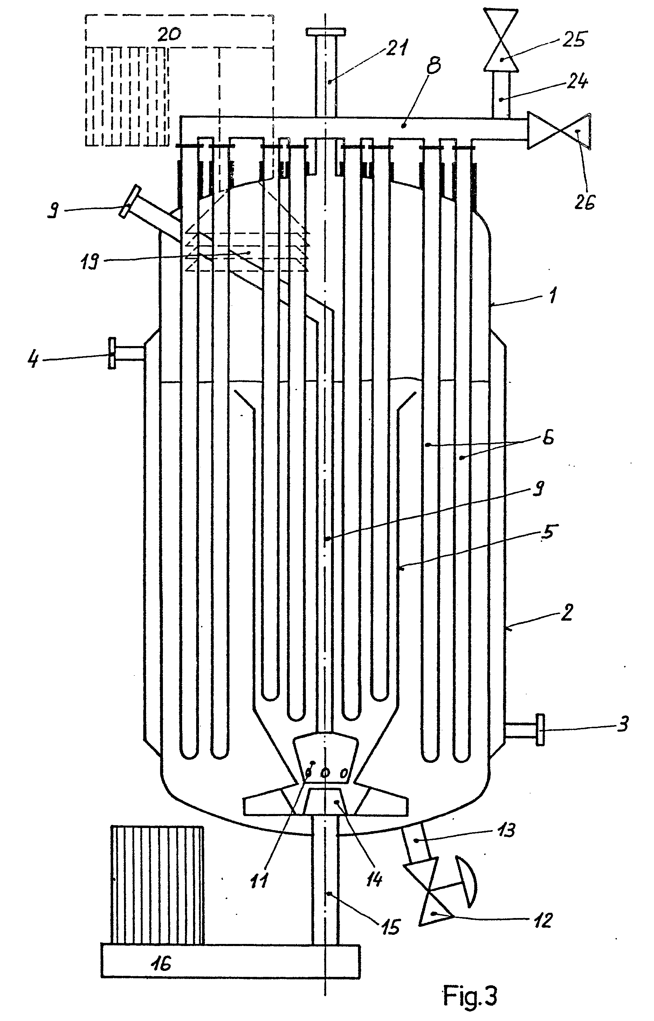

Fig. 3 zeigt den Längsschnitt durch einen Fermenter, dessen Volumen weitgehend mit Membranfiltern 6 ausgefüllt ist. Neben den im Leitrohr 5 befindlichen Membranfiltern ist auch der Raum zwischen Fermenterwand 1 und Leitrohr 5 ausgefüllt. Die Membranfilter 6 ragen in ihrem oberen Teil über den Deckel des Fermenters hinaus und münden zur Aufnahme des Permeates in dem darüber angebrachten Sammelrohr 8. Das Sammelrohr 8 ist mit einem Stutzen 24 und einem Ventil 25 versehen, über welche die Membranfilter rückgespült werden können. Beim Rückspülen ist das Ventil 26 geschlossen.

Im Betrieb des kontinuierlich arbeitenden Fermenters wird Substrat unter mässigem Ueberdruck oder durch die Selbstansaugung der Umwälzturbine 14, zur Ueberwindung des im Fermenter vorgesehenen Druckes, über die Rohrleitung 7 dem Fermenterkessel 1 zugeführt. Die Hauptmenge der erforderlichen Begasungsluft wird über die Rohrleitung 9 und den Verteilkörper 11 direkt der Umwälzturbine 14 zugeführt. Die Umwälzturbine 14 wird durch den Elektromotor 16 über die Welle 15 angetrieben. Gleichzeitig mit der Substratzufuhr wird, ohne Unterbruch, Luft oder mit Sauerstoff angereicherte Luft über die Leitung 17 und die Rohrstücke 18 in die Rohre 6 der Membranfilter eingeführt. Die Luft wird von der durch die Umwälzturbine 14 angesaugen Nährsubstratlösung, die die metabolisierenden Mikroorganismen in Suspension enthält, nach unten mitgerissen und gewährleistet schon auf dem Wege zur Turbine 14 eine ausreichende Belüftung. Die im Abwärtsstrom befindlichen Luftblasen werden durch die Umwälzturbine 14 in bekannter Weise weiter zerkleinert und bewirken zusammen mit der Luft aus dem Verteilerkörper 11 eine sehr intensive Belüftung des Substrates und somit der Mikroorganismen. Gleichzeitig fliesst durch die Rohrmembranen 6′, deren Porendurchmesser kleiner als die kleinsten am Stoffwechsel beteiligten Mikroorganismen ist und letztere daher nicht hindurchtreten können, das gewünschte Produkt. Das Permeat sammelt sich in den Zwischenräumen der Membranfilterrohre 6 und verlässt über die Rohrleitung 8 den Fermenter. Der Fermenter ist mit einer Druckregulierung in Form eines Kontaktmanometers 23 versehen, welches das Abgasventil 22 so steuert, dass im Fermenterkopf ein ständiger Ueberdruck eingestellt werden kann, der dazu dient, die Durchflussrate durch das Membranfilter zu regulieren und damit den biologischen Erfordernissen anzupassen. Das Steuerventil 22 ist in der Abgasleitung 21 des mechanischen Schaumabscheiders 19 angebracht, der selbst durch einen Elektromotor 20 angetrieben wird. Eine nicht gezeigte Gewichtsregulierung ermöglicht es, das Verhältnis zwischen rezirkulierenden Mikroorganismen und der erforderlichen optimalen Konzentration zu kontrollieren. Das in der Abflussleitung 13 angebrachte Ernteventil erlaubt die Entnahme bei unerwünschtem Ansteigen der Mikroorganismenkonzentration.

Eine Reinigung der Membranen kann auch durch Rückspülen der Membranen über die Rückspülleitung 24 bei geöffnetem Ventil 25 und geschlossenem Ablaufventil 26 erfolgen. Diese Rückspülung kann mit Permeat auch während der Fermentation in erforderlichen zeitlichen Abständen durchgeführt werden.

Ein besonderer Vorteil des erfindungsgemässen Fermenters besteht darin, dass durch den Einbau von Membranfiltern im Innern des Fermenters eine kontinuierliche Arbeitsweise unter sterilen Bedingungen erst möglich wird. Neben der schädlichen Wirkung der einleitend genannten Pumpen, Rohrleitungen und Messonden beim Aufbau eines externen Systems, entfallen erfindungsgemäss zusätzliche Investitionen für diese Einrichtungen.

Ein weiterer Vorteil ist, dass die Kühlung eines konventionellen Membranfiltersystems entfällt, worin die freiwerdende Wärme der erforderlichen Umwälzpumpe eines externen Membranfilters extra abgeführt werden müsste.

Ein entscheidender Vorteil liegt auch bei der Fermentierung mit auf Sauerstoffmangel empfindlich reagierenden Mikroorganismen. Diese können durch den erfindungsgemässen Fermenter gleichzeitig, unter Abtrennung der Stoffwechselprodukte, ohne Unterbruch weiter belüftet werden. Die Mikroorganismen werden so in keinem Stadium aus ihrem Milieu entfernt, an welches sie sich gewöhnt haben.

Als bevorzugte Anwendungsgebiete haben sich die Herstellung von Essig, Alkohol, Antibiotika, organischen Säuren, insbesondere Citronensäure erwiesen.

## Ansprüche

1. Vorrichtung zur aeroben Züchtung von Mikroorganismen, unter gleichzeitiger Abtrennung und Entfernung der entstehenden Stoffwechselprodukte mittels Membranfiltration im Inneren eines zylindrischen Fermenters, dadurch gekennzeichnet, dass feststehende Membranfilter (6) im Innern eines zentralen Leitrohres (5) des Fermenters (1) angebracht sind.

2. Vorrichtung nach Anspruch 1, dadurch gekennzeichnet, dass die Membranfilter (6) aus Rohren bestehen.

3. Vorrichtung nach den Ansprüchen 1 und 2, dadurch gekennzeichnet, dass Luftzufuhrrohre (18) in die Rohre (6) der Membranfilter einmünden.

4. Vorrichtung nach den Ansprüchen 1 bis 3, dadurch gekennzeichnet, dass ein Ventil (22) zur Druckregelung im Fermenter in der Abgasleitung (21) eines mechanischen Schaumabscheiders (19) angebracht ist.

5. Vorrichtung nach den Ansprüchen 1 bis 4, dadurch gekennzeichnet, dass die Permeatleitung (8) der Membranfilter (6) mit einem Anschluss zur Rückspülung während der Fermentation versehen ist.

## Claims

1. Equipment for the aerobic cultivation of microorganisms with simultaneous separation and withdrawal of the metabolic products by diaphragm-filtration inside a cylindrical fermenter, wherein the diaphragm filters (6) are firmly located inside the central draft tube (5) of the fermenter (1).

2. Equipment as defined in claim 1, wherein said diaphragm filters (6) are formed as tubes.

3. Equipment as defined in claims 1 and 2, wherein air supply conduits (18) are extending into the pipes (6) of the diaphragm filters.

4. Equipment as defined in claims 1 to 3, wherein is located a valve (22) for the control of the pressure inside the fermenter in the exhaust conduit (21) of a mechanical foam breaking device (19).

5. Equipment as defined in claims 1 to 4, wherein the conduit (8) for collecting a permeate of the diaphragm filters (6) is provided with a connecting line for flushing back during the fermentation.

## Revendications

1. Appareil de fermentation pour la culture aérobie de microorganismes avec séparation et évacuation simultanées des produits de métaboli- sation, au moyen d'un filtre à membrane (6) disposé à l'intérieur dudit fermenteur cylindrique caractérisé en ce que du filtre à membrane (6) est disposé à l'intérieur d'un conduit central (5) dudit fermenteur.

2. Appareil selon les revendications 1 et 2, caractérisé en ce que les filtres à membrane (6) sont constitués par des tubes.

3. Appareil selon les revendications 1 et 2 caractérisé en ce que des conduits (18) d'amenée d'air débouchent dans les tubes (6) des filtres à membrane.

4. Appareil selon les revendications 1 à 3, caractérisé en ce qu'une soupape de réglage de la pression (22) est disposée en fermenteur dans le conduit d'évacuation de gaz (21) d'un séparateur mécanique de mousse (19).

5. Appareil selon les revendications 1 à 4, caractérisé en ce que le conduit d'évacuation du perméat (8) le filtre à membrane (6), est pourvu d'un raccord pour rinçage de retour pendant la fermentation.

Fig. 1

0 007 133

Fig. 2

2

Fig.3